# EUROPEAN PATENT SPECIFICATION

(11) **EP 4 054 522 B1**
(45) Date of publication and mention of the grant of the patent: **21.10.2026**
(21) Application number: 20807914.5
(22) Date of filing: 05.11.2020
(51) Int. Cl.: A61K 9/00, A61K 31/192, A61K 47/10, A61P 3/00

(54) **PHARMACEUTICAL LIQUID COMPOSITION, KIT OF PARTS COMPRISING THE PHARMACEUTICAL LIQUID COMPOSITION, AND METHOD FOR PREPARING THE PHARMACEUTICAL LIQUID COMPOSITION**
PHARMAZEUTISCHE FLÜSSIGE ZUSAMMENSETZUNG, SATZ VON TEILEN MIT DER PHARMAZEUTISCHEN FLÜSSIGEN ZUSAMMENSETZUNG UND VERFAHREN ZUR HERSTELLUNG DER PHARMAZEUTISCHEN FLÜSSIGEN ZUSAMMENSETZUNG
COMPOSITION LIQUIDE PHARMACEUTIQUE, KIT DE PIÈCES COMPRENANT LA COMPOSITION LIQUIDE PHARMACEUTIQUE, ET PROCÉDÉ DE PRÉPARATION DE LA COMPOSITION PHARMACEUTIQUE LIQUIDE

(30) Priority: 05.11.2019 NL 2024160
(43) Date of publication of application: 14.09.2022
(73) Proprietor: Mperium B.V., 1244 RL Ankeveen (NL)
(72) Inventor: MEIJLINK, Peter, 1244 RL Ankeveen (NL)
(74) Representative: Arnold & Siedsma
(86) International application number: PCT/NL2020/050694
(87) International publication number: WO 2021/091382

(56) References cited:
- MICHAEL L MISEL ET AL: "Sodium Benzoate for Treatment of Hepatic Encephalopathy", GASTROENTEROLOGY & HEPATOLOGY VOLUME, vol. 9, 1 April 2013 (2013-04-01), XP055267026
- ANOMYNOUS: "Application Number 20-645 Medical Review FDA", 15 February 2005 (2005-02-15), pages 1 - 55, XP055260195, Retrieved from the Internet <URL:http://www.accessdata.fda.gov/drugsatfda_docs/nda/2005/020645s000_MedR.pdf> [retrieved on 20160322]
- GREGORY M ENNS ET AL: "Survival after Treatment with Phenylacetate and Benzoate for Urea-Cycle Disorders A BS T R AC T", THE NEW ENGLAND JOURNAL OF MEDICINE, vol. 356, no. 22, 31 May 2007 (2007-05-31), pages 2282 - 2292, XP055148817

## Description

### Field of Invention

The field of the invention relates to a pharmaceutical liquid composition. The field of the invention further relates to a kit of parts comprising the pharmaceutical liquid composition. The field of the invention further relates to a method for preparing the pharmaceutical liquid composition according to the invention. The field of the invention further relates to a pharmaceutical liquid composition for use as a medicament.

### Background

Generally known is a use of sodium benzoate as an alternative for present therapeutic treatments of certain disorders of the urea cycle. Urea cycle disorders can result from decreased activity of any of the following enzymes: N-acetylglutamate synthetase (NAGS), carbamyl phosphate synthetase (CPS), argininosuccinate synthetase (ASS), ornithine transcarbamylase (OTC), argininosuccinate lyase (ASL), or arginase (ARG).

Sodium benzoate is a metabolically active compound that can serve as alternative to urea for the excretion of waste nitrogen. Preceded by acylation, benzoate conjugates with glycine to form hippuric acid, which is rapidly excreted by the kidneys by glomerular filtration and tubular secretion. One mole of hippuric acid contains one mole of waste nitrogen. Thus, one mole of nitrogen is removed per mole of benzoate when it is conjugated with glycine.

On admission to the hospital, patients with hyperammonemia and a suspected or confirmed urea cycle disorder (UCD) diagnosis may be treated with a pharmaceutical liquid composition known as Ammonul, which is a solution of sodium phenylacetate and sodium benzoate. Sodium benzoate and sodium phenylacetate are given in parallel in severe acute situations. The solution is a concentrated solution and must be diluted before intravenous administration via a central venous catheter. Administration through a peripheral intravenous catheter may cause burns. Ammonul may not be administered by any other route. It is prescribed that the Ammonul is sterilized before use and diluted with sterile 10% Dextrose Injection before administration.

However, a need exists to provide a pharmaceutical composition containing sodium benzoate as active ingredient, which may be used daily by patients for treating disorders of the urea cycle without the active intervention of a physicist. Moreover, a need exists to administer the pharmaceutical composition orally, wherein a dosage for administration can easily be adapted according to the personal requirements. The dosage is determined by weight for neonates, infants and young children, and by body surface area for larger patients, including older children, adolescents, and adults. As such, the dosage needs to be adapted to the personal characteristics.

When sodium benzoate is compounded into capsules various capsules would be needed to provide the requirement dosage for each patient. Moreover, capsules are not easily swallowed by patients.

Another form to provide sodium benzoate is by dissolving a powder of sodium benzoate in water. The powder dosage may be measured using differently sized measuring spoons, which may lead to an imprecise dosage level. The imprecise dosing measurement, and the need to mix the powder with a fluid for administration, leads to a lack of compliance in taking the prescribed dose at the required intervals. Moreover, contact with the powder is discouraged because it is considered potentially hazardous in the event of skin contact (irritant), eye contact (irritant), or inhalation. Additionally, a solution of sodium benzoate in water has been found to have a bitter taste, which makes it difficult to swallow the solution by patients.

MICHAEL L MISEL ET AL: "Sodium Benzoate for Treatment of Hepatic Encephalopathy",GASTROENTEROLOGY & HEPATOLOGY VOLUME, Part 9, 1 April 2013 (2013-04-01), describes the use of Ammonul, which is a solution of sodium phenylacetate and sodium benzoate, and describes the use of a powder of sodium benzoate to be measured and mixed into a flavored beverage or food supplement.

Thus, a need exists to provide a pharmaceutical composition containing sodium benzoate as active ingredient, which pharmaceutical composition may be used daily and stored by a patient in a simple way.

### Summary

Embodiments of the invention aim to provide a pharmaceutical composition the sodium salt of benzoic acid and/or the potassium salt of benzoic acid as the active ingredient, wherein the pharmaceutical liquid composition may be used daily and stored by a patient in a simple way.

Particular embodiments aim to provide a pharmaceutical liquid composition comprising the sodium salt of benzoic acid and/or the potassium salt of benzoic acid as the active ingredient, wherein the pharmaceutical liquid composition provides a user friendly and precise control on dosing of the sodium salt of benzoic acid and/or the potassium salt of benzoic acid for oral administration.

Particular embodiments aim to provide a pharmaceutical liquid composition comprising the sodium salt of benzoic acid and/or the potassium salt of benzoic acid as the active ingredient, wherein the pharmaceutical liquid composition helps a patient to maintain compliance with their dosing regimen.

Particular embodiments aim to provide a pharmaceutical liquid composition comprising the sodium salt of benzoic acid and/or the potassium salt of benzoic acid as the active ingredient, wherein the pharmaceutical liquid composition is usable to administer in a way that supports a swallowing by children and/or by patients having swallowing problems.

According to a first aspect of the invention there is provided a pharmaceutical liquid composition comprising a solution of the sodium salt of benzoic acid and/or the potassium salt of benzoic acid, preferably the sodium salt of benzoic acid or the potassium salt of benzoic acid, in an aqueous medium at a concentration of at least 1.39 mmol/ml (200 mg/ml for SB);

Wherein the aqueous medium comprises:
a. glycerol having a volume concentration of at least 1.0 ml/l relative to the total volume of the aqueous medium;
b. a viscosity enhancing agent comprising a cellulose derivate being selected from the group consisting of methyl-ethyl-propyl cellulose, hydroxypropylcellulose, hydroxyethylcellulose, sodium carboxymethyl cellulose, microcrystalline cellulose; and
c. water;
and wherein the pH of the liquid composition is in the range between 6.0 and 10.0, and wherein the weight ratio between the glycerol and the viscosity enhancing agent is between 200 : 1 and 10 : 1.

The pharmaceutical liquid composition has a concentration level of the sodium salt of benzoic acid and/or the potassium salt of benzoic acid of at least 1.39 mmol/ml (200 mg/ml SB). The concentration level of the sodium salt of benzoic acid and/or the potassium salt of benzoic acid supports the use of a lower amount of solution while providing a higher amount of the salt of benzoic acid.

Additionally, the inventors have found that the concentration level of the sodium salt of benzoic acid and/or the potassium salt of benzoic acid of at least 1.39 mmol/ml surprisingly provides microbiological stability of the pharmaceutical liquid composition. The pH of the pharmaceutical liquid composition is in the range between 6.0 and 10.0. The pH level is primarily determined by the concentration of the sodium and/or potassium salt of benzoic acid, such as sodium benzoate, in the solution.

Bacteria Yeasts and fungi thrive within the pH range of 5.5 to 8.0 in aqueous media. Known is that a very high or very low pH can make a product more hostile for some microbes. If the pH is high enough, for example, over pH 10, a preservative may not be needed.

Sodium benzoate is a food grade preservative. It is soluble in water where it converts to benzoic acid, its active form, at a low pH. Sodium benzoic acid is very active at pH 3.0 (94%), while it is known to substantially completely loose its activity when increasing pH towards 6.0. The effectiveness of sodium benzoate as a preservative at decreasing pH (increasing acidity) is because the ratio of undissociated (i.e., free) benzoic acid to ionized benzoic acid increases as the pH decreases. It is generally accepted that the undissociated benzoic acid is the active antimicrobial agent.

Without being bound to theory it is believed that for the sodium salt of benzoic acid and/or the potassium salt of benzoic acid undissociated benzoic acid is in equilibrium to the salt of benzoic acid in the solution according to the following equation:

C₆H₅COOM + H₂O <=> C₆H₅COOH + MOH

wherein M is the positive counterion of the benzoate, which is sodium or potassium.

As the pKa is about 4, it can be derived that at pH 7 about 99.9% is benzoate and about 0.1 % is benzoic acid. The undissociated benzoic acid is believed to be the active form for preservative effect.

Thus, the results of the present invention unexpectedly show that the concentration level of the sodium salt of benzoic acid and/or the potassium salt of benzoic acid of at least 1.39 mmol/ml contribute to a high microbiological stability of the pharmaceutical liquid composition.

It has now been found by the inventors that the pharmaceutical liquid composition as specified in claim 1 provides microbiological stability of the pharmaceutical liquid composition even when the pH of the pharmaceutical liquid composition is higher than 6.0.As such, the salt of the benzoic acid is self-preservative in the pharmaceutical liquid composition and no additional preservative is needed to provide a microbiological stable solution.

Furthermore, without being bound by theory, the use of glycerol further is assumed to support the microbiological stability of the pharmaceutical liquid composition.

The combination of the use of glycerol and the concentration level of the sodium salt of benzoic acid and/or the potassium salt of benzoic acid of at least 1.39 mmol/ml surprisingly provides a high microbiological stability of the pharmaceutical liquid composition. Said level of microbiological stability is higher than can be attributed to the use of glycerol alone.

For mixtures of glycerol in water it is generally known that a very high concentration, e.g. at least 40 vol-%, of glycerol in water is needed to obtain a microbiological stability, in particular a bacteriostatic and/or bactericidal property.

Thus, the results of the present invention show that the concentration level of sodium salt of benzoic acid and/or the potassium salt of benzoic acid of at least 1.39 mmol/ml contribute to a high microbiological stability of the pharmaceutical liquid composition.

A concentration of the sodium salt of benzoic acid and/or the potassium salt of benzoic acid in the aqueous medium (expressed in mmol/ml) according to the invention is determined by measuring the concentration of benzoic acid in mmol/ml using High-performance liquid chromatography (HPLC) technique. HPLC techniques to determine the concentration of benzoic acid in an aqueous medium are generally known. The HPLC technique may be optimized to separate the benzoic acid from the other components of the pharmaceutical liquid composition depending on the composition of the pharmaceutical liquid composition.

For the calculation of the concentration of the sodium salt of benzoic acid and/or the potassium salt of benzoic acid in the aqueous medium (expressed in mmol/ml) it may be determined that the benzoic acid is substantially completely dissolved in the aqueous medium based on the salt form. Thus, the concentration of the sodium salt of benzoic acid and/or the potassium salt of benzoic acid in the aqueous medium may be assumed to be substantially equal to the concentration of benzoic acid in the aqueous medium (in mmol/ml).

A concentration of a sodium salt of benzoic acid according to the invention, when being used to form the pharmaceutical liquid composition, may be calculated as a concentration expressed in mg/ml based on the determined amount of the sodium salt of benzoic acid and/or the potassium salt of benzoic acid expressed in mmol/ml and based on the molar mass of sodium benzoate (i.e. 144.1 g/mol).

Liquid formulations such as solutions/drops dosage forms are rated as the dosage form of choice for infants already from their first month of life. One important consideration with liquid formulations is the dose volume, large volumes are inconvenient for patients and care takers. Usually, dose volumes below 5 ml are suitable for children under the age of 5 and volumes larger 10ml for children of 5 years and older (EMEA 2000). The pharmaceutical liquid composition according to the invention supports an easy swallowing by children and/or patients having swallowing problems.

Additionally, the combination of the glycerol and the viscosity enhancing agent provide a precise control on dosing of the salt of benzoic acid for oral administration due to an adequate control on the viscosity of the pharmaceutical liquid composition. The viscosity enhancing agent controls the viscosity of the pharmaceutical liquid composition at a level which is suitable for precise dosing of the pharmaceutical liquid composition, especially for a precise dosing of the pharmaceutical liquid composition in the range of 0.5 ml - 20.0 ml per dose. In particular, a precise dosing of the pharmaceutical liquid composition is critical for infants, which require a target dosing of the active ingredient less than less than 5.0 grams per day, often between 1.0 and 5.0 grams per day depending on age, wherein the daily amount may be divided in 4 separate doses per day.

The use of the glycerol as solvent together with water provides that the viscosity enhancing agent controls the viscosity of the pharmaceutical liquid composition, while the solution stability of the salt of benzoic acid in the pharmaceutical liquid composition is not adversely affected. As a result, the concentration of the salt of benzoic acid in pharmaceutical liquid composition remains stable over a longer time.

Additionally, the glycerol supports a more palatable pharmaceutical liquid composition. It has surprisingly been found that the glycerol provides a sweetening effect to the pharmaceutical liquid composition, which masks a salty taste of the benzoate solution. Thus, the glycerol supports swallowing of the pharmaceutical liquid composition.

In an embodiment, the pH of the liquid composition is in the range of 7.0 to 9.0, more preferably in the range of 7.0 to 8.0. The pharmaceutical liquid composition surprisingly has microbiological stability at pH higher than 7.0. As such, the salt of the benzoic acid is self-preservative in the pharmaceutical liquid composition at said pH and no additional preservative is needed to provide a microbiological stable solution, even at a pH higher than 7.0.

The glycerol has a volume concentration of at least 1.0 ml/l to the total volume of the aqueous medium, preferably in the range 5.0 ml/l to 20.0 ml/l relative to the total volume of the aqueous medium.

1.0 ml/l corresponds to 1.0 ml of glycerol in 1 liter total volume of the aqueous medium. 20.0 ml/l corresponds to 20 ml of glycerol in 1 liter total volume of the aqueous medium.

The aqueous medium containing glycerol has a glycerol concentration of at least 1.0 ml/l to provide a suitable co-solvent for the viscosity enhancing agent. The amount of glycerol concentration is preferably less than 20.0 ml/l as a higher concentration of glycerol may have an adverse affect on stomach or intestines of the patient, such as diarrhea and flatulence.

At concentrations of 5.0 to 10.0 ml/l, it is found that glycerol in a water-based solution possesses unexpected bacteriostatic and fungicidal properties.

The viscosity enhancing agent comprises a cellulose derivate, the cellulose derivate being selected from the group consisting of methyl-ethyl-propyl cellulose, hydroxypropylcellulose, hydroxy ethyl cellulose, sodium carboxymethyl cellulose, microcrystalline cellulose.

In a particular embodiment, the viscosity enhancing agent comprises hydroxyethylcellulose. It has been found that hydroxyethylcellulose may suitably control the viscosity of the pharmaceutical liquid composition at a low concentration of the viscosity enhancing agent, while not adversely affecting the solution stability of the sodium salt of benzoic acid and/or the potassium salt of benzoic acid.

The weight ratio between glycerol and the viscosity enhancing agent is between 200 : 1 and 10 : 1. The weight ratio is relevant for providing a suitable solvent for the viscosity enhancing agent both during preparation of the pharmaceutical liquid composition and the storage of the pharmaceutical liquid composition to obtain the desired viscosity in the aqueous medium.

The sodium salt of benzoic acid or the potassium salt of benzoic acid has a good solubility in the aqueous medium.

Sodium benzoate may be obtained in relatively pure form by the reaction of benzoic acid with sodium hydroxide. Potassium benzoate may be obtained in a relatively pure form by oxidizing toluene to benzoic acid followed by a neutralization with potassium hydroxide.

A concentration of a potassium salt of benzoic acid according to the invention, when being used to form the pharmaceutical liquid composition, may be calculated as a concentration expressed in mg/ml based on the determined amount of the sodium salt of benzoic acid and/or the potassium salt of benzoic acid expressed in mmol/ml and based on the molar mass of potassium benzoate (i.e. 160.0 g/mol).

In an embodiment, the sodium salt of benzoic acid and/or the potassium salt of benzoic acid has a concentration in the range of 1.39 mmol/ml (200 mg/ml SB) to 5.20 mmol/ml (750 mg/ml SB), preferably in the range of 1.39 mmol/ml to 3.47 mmol/ml (500 mg/ml SB), more preferably in the range of 1.56 mmol/ml (225 mg/ml SB) to 2.78 mmol/ml (400 mg/ml SB).

The lower limit is determined by the effect of the concentration of the sodium salt of benzoic acid and/or the potassium salt of benzoic acid on the microbiological stability of the pharmaceutical liquid composition.

The higher limit is mainly determined by the solution stability of the pharmaceutical liquid composition. In case a higher concentration is selected, a solution stability of the sodium salt of benzoic acid and/or the potassium salt of benzoic acid may become less.

It has been found that a concentration of the sodium salt of benzoic acid and/or the potassium salt of benzoic acid of higher than 1.39 mmol/ml (200 mg/ml SB), preferably higher than 1.56 mmol/ml (225 mg/ml SB) , more preferably in the range of 1.39 mmol/ml (200 mg/ml SB) to 2.78 mmol/ml (400 mg/ml SB), raises the pH of the pharmaceutical liquid composition higher than 6.0, preferably higher than 7.0, more preferably in the range of 7.0 to 8.0.

A sodium salt of benzoic acid at 1.73 mmol/ml (i.e. 250 mg/ml SB) in an aqueous solution was confirmed to be in alkaline regions, > 7. The alkaline solution pH limited selection of effective and regulatory acceptable preservatives systems. Application of parabens may be rejected due to intended pediatric use and from a regulatory perspective (parabens are suspected allergen). Unexpectedly a solution of sodium salt of benzoic acid of 1.39 mmol/ml (200 mg/ml SB) and higher has been found to be self-preservative without using additional preservative agents. The advantage is that no additional preservative agents need to be added to the pharmaceutical liquid composition.

In an embodiment, the viscosity enhancing agent has a concentration of at least 0.001 g/ml relative to the total volume of the aqueous medium, preferably in the range 0.001 g/ml to 1.000 g/ml relative to the total volume of the aqueous medium, more preferably in the range 0.001 g/ml to 0.100 g/ml relative to the total volume of the aqueous medium. The relatively low amount of viscosity enhancing agent has been found suitable for raising the viscosity, while not adversely affecting the solution stability of the sodium salt of benzoic acid and/or the potassium salt of benzoic acid.

In an embodiment, the viscosity of the liquid composition is in the range of 100 mPa.s - 500 mPa.s at 20 °C. It has been found that the viscosity of the liquid composition, which is higher than of water or glycerol at 20 °C, enhances a precise dosing of the pharmaceutical liquid composition. The pharmaceutical liquid composition may be administered using common syringes suitable for a dose in the range of 1.0 ml - 20 ml. The accuracy of dosing is more critical for a dose in the range of 1.0 ml - 5.0 ml. The accuracy of dosing the pharmaceutical liquid composition is in the range of +/- 5% (at a low volume around 0.5 - 2.5 ml) or even better (at volumes larger than 2.5 ml).

In an embodiment, the pharmaceutical liquid composition comprises an additional sweetening agent, which comprises a natural sweetening agent and / or an artificial sweetening agent.

Natural sweetening agents may be present in a range of 0.01 % w/v to 1.00 % w/v with respect to the total volume of the aqueous medium, preferably in the range of 0.01 % w/v to 0.10 % w/v with respect to the total volume of the aqueous medium. Artificial sweeteners may be present in a range of 0.01 % w/v to 1.00 % w/v with respect to the total volume of the aqueous medium, preferably in the range of 0.01 % w/v to 0.10 % w/v with respect to the total volume of the aqueous medium.

In an embodiment, the natural sweetener is selected from the group consisting of sucrose, dextrose, fructose, sorbitol, xylitol, vanilla, mannitol and mixtures thereof and wherein the artificial sweetener is selected from the group consisting of sodium saccharine, sodium cyclamate, aspartame, lacititol, isomalt, sucralose acesulfame K, neohespiridine, sucralose and mixtures thereof.

In an embodiment, the sweetening agent comprises aspartame having a concentration of at least 0.01 g/ml relative to the total volume of the aqueous medium, preferably in the range of 0.01 - 0.10 g/ml.

In an embodiment, the sweetening agent comprises sucrose having a concentration of at least 0.01 g/ml relative to the total volume of the aqueous medium, preferably in the range of 0.01 - 0.10 g/ml.

In an embodiment, the sweetening agent comprises aspartame and sucrose and wherein the concentration of aspartame relative to the concentration of sucrose is in the range 1: 2 to 2 : 1. The combination of the aspartame and sucrose in said ratio provides an enhanced sweetening effect. In particular, the sweetening is provided both for the short term taste and longer term aftertaste.

In an embodiment, the viscosity enhancing agent comprises a hydroxyethylcellulose having a weight average molecular weight in the range of 200.000 - 2.000.000 Da.

According to another aspect of the invention there is provided a kit of parts comprising:
a. a pharmaceutical liquid composition according to the present invention; and
b. a flavouring liquid concentrate, the flavouring liquid concentrate comprising at least one flavouring agent.

The kit of parts provides a combination of the pharmaceutical liquid composition of the invention and the flavouring liquid concentrate. The flavouring liquid concentrate can be administered in drops, such as administered in drops to a mixture containing water and the pharmaceutical liquid composition to obtain a flavoured pharmaceutical liquid mixture.

A flavouring liquid concentrate according to the present invention is liquid at room temperature. As such, the flavouring liquid concentrate can easily be administered in drops. When applying one drop to three drops, the amount of flavouring liquid concentrate, which is added to the pharmaceutical liquid composition, is in the range of 0.010 g - 0.150 g.

The flavour of a poor tasting pharmaceutical ingredient agent in a liquid dosage form is inevitably detected during the drinking process or immediately after swallowing.

Therefore, it is important to mask the often disagreeable taste or aftertaste of the active ingredient during the time that the medicine is in the mouth and/or after administration. This invention relates to a kit of taste-masking platform compositions wherein the taste and/or aftertaste of a pharmaceutical active ingredient is reduced to an acceptable level. The flavouring liquid concentrate enhances an easy swallowing of the pharmaceutical liquid composition.

The at least one flavouring liquid concentrate may be used to improve the taste by masking a bad taste of the pharmaceutical liquid composition. As the pharmaceutical liquid composition already contains the sweetening agent glycerol, the at least one flavouring liquid concentrate may be primarily comprised of flavouring agents. The flavouring agents may be highly concentrated extracts of natural flavouring compositions or may be synthetic flavouring agent.

A flavouring agent in the context of the invention comprises a flavor essence or flavor component. Additionally, the flavouring agent may contain a carrier fluid which is configured for stabilizing the flavor essence or flavor component. The carrier fluid may in examples be a glycol, such as propylene glycol, an alkyl alcohol, an aryl alcohol and mixtures thereof. Generally, the carrier fluid may be selected to maintain a stability of the flavor essence or flavor component for at least 6 months at room temperature.

The combination of the sweetening agent contained in the pharmaceutical liquid composition and the flavouring agents of the flavouring liquid concentrate provide the taste masking effect.

The at least one flavouring liquid concentrate may easily mix and dissolve in the pharmaceutical liquid composition or in a mixture containing water and the pharmaceutical liquid composition. As the sweetening agent is already dissolved in the pharmaceutical liquid composition, a mixture of water, the pharmaceutical liquid composition and at least one flavouring liquid concentrate is ready to be used directly after adding drops of flavouring liquid concentrate.

A mixture containing water and the pharmaceutical liquid composition may be prepared by mixing an amount of water and an amount of pharmaceutical liquid composition. The mixing ratio between pharmaceutical liquid composition and water may be in the range of 1:1 - 1:20, preferably in the range of 1:2 - 1:10. For example, a 1.0 ml pharmaceutical liquid composition may be mixed with 5.0 ml water (1: 5) or may be mixed with 10.0 ml water (1: 10).

The amount of the at least one flavouring liquid concentrate can be suitably selected to adjust the flavor of the pharmaceutical liquid mixture to obtain a personalized medication for the user. Moreover, as the flavouring liquid concentrate does not negatively affect the taste (based on the to effect of maintaining the pH of the resulting mixture higher than 5.5) the patient may freely select the amount of flavouring liquid concentrate without running into disappointing and misunderstood taste affects (at higher amounts of flavouring liquid concentrate).

The flavoured pharmaceutical liquid mixture provides improved personal palatable use to children and patients and may support children and patients to maintain compliance with their dosing regimen.

In an embodiment, the flavouring liquid concentrate comprises a first flavouring agent and a second flavouring agent, wherein the first flavouring agent is selected from the group of mint, ginger, caramel, liquorice, peppermint and eucalyptol, and mixtures thereof. Each of mint, ginger, caramel, liquorice, peppermint and eucalyptol may have a positive effect on reducing bitterness of the pharmaceutical liquid composition of the invention.

Further disclosed herein is that the first flavouring agent may have a concentration in the range of 2- 80 weight-%, preferably 2 - 60 weight-%, of the total weight of the flavouring liquid concentrate.

Disclosed herein is that the second flavouring agent is a fruit essence, including blackcurrant, apple, pear, peach, berry, wildberry, date, blueberry, kiwi, strawberry, raspberry, cherry, plum, pineapple, mango, apricot and citrus oils such as lemon, orange, lime and grapefruit oils.

In a particular embodiment, the flavouring liquid concentrate comprises a first flavouring agent and a second flavouring agent, wherein the first flavouring agent is selected from the group of mint, ginger, caramel, liquorice, peppermint and eucalyptol, and mixtures thereof, and wherein the second flavouring agent is blackcurrant.

In a particular embodiment, the flavouring liquid concentrate, when dissolved in purified water at a concentration of 0.10 g / 5 ml water, has a pH of at least 5.5.

It has surprisingly been found by the inventors that the flavouring liquid concentrate effectively masks the bitterness and/or salty taste of the pharmaceutical liquid composition of the salt of benzoic acid, when the flavouring liquid concentrate, when dissolved in purified water at a concentration of 0.10 g / 5 ml water, has a pH of at least 5.5. Flavouring liquid concentrates, which potentially may reduce the bitterness and/or salty taste have found not to improve the taste, but to deteriorate the taste, when the flavouring liquid concentrate lowers the pH when added to the pharmaceutical liquid composition of the salt of benzoic acid. In particular, a blackcurrant flavour agent has been found to provide a flavouring liquid concentrate, which supports the requirement of maintaining the pH above 5.5 (using the above mentioned test method).

In order to determine, how the flavouring liquid concentrate affects the taste of the pharmaceutical liquid composition of the salt of benzoic acid according to the invention, it has been established that a taste perception test method can be used for flavouring liquid concentrates, wherein the flavouring liquid concentrate is dissolved in purified water (e.g. demi water) at a concentration of 0.10 g / 5 ml water. It has been found that, using this test method, when the pH is at least 5.5 in 5 ml water, the taste can effectively be improved (i.e. bitterness is improved).

Purified water is to be understood as demi water that has been mechanically filtered or processed to remove impurities and make it suitable for use. Purified water has a pH in the range 6.0 - 6.5.

In a particular embodiment, the flavouring liquid concentrate, when dissolved in purified water at a concentration of 0.25 g / 5 ml water, has a pH of at least 6.0. This further enhances the consistency of the taste effect of the flavouring liquid concentrate.

The flavouring liquid concentrate may be substantially free of a sweetening agent.

As the pharmaceutical liquid composition may comprises an effective amount of sweetening agents, such as glycerol, for sweetening the pharmaceutical liquid composition, the flavouring liquid concentrate may be substantially free of a sweetening agent.

In particular, the at least one flavouring liquid concentrate may consist essentially of flavouring agents. Sweetening agents are primarily solid at room temperature and need a sufficient amount of solvent to provide a liquid solution state. Adding a sweetening agent to the flavouring liquid concentrate would require a bigger amount of solvent to provide and maintain a liquid solution state.

In an embodiment, each flavouring agent has a concentration in the range of 2 - 100 weight-% of the total weight of the flavouring liquid concentrate. In a particular embodiment, the sum of the at least one flavouring agent is in the range of 80 - 100 weight-% of the total weight of the flavouring liquid concentrate.

In an embodiment, a flavouring liquid concentrate comprises a first flavouring agent and a second flavouring agent. A combination of the first flavouring agent and the second flavouring agent may provide a better taste, for instance by combining several beneficial tastes.

In an embodiment, the kit comprises a medical dropper for administering drops of the at least one flavouring liquid concentrate. The medical dropper may be used to administer drops of the at least one flavouring liquid concentrate. Preferably, each of the flavouring liquid concentrates is contained in a small container, such as a bottle, wherein the bottle further is provided with a medical dropper.

The at least one flavouring agent may be selected from the group consisting of anise oil, cinnamon oil, vanilla, vanillin, ginger, cocoa, chocolate, caramel , menthol, grape, mint, peppermint oil, oil of wintergreen, clove oil, bay oil, anise oil, liquorice, thyme oil, cedar leaf oil, oil of nutmeg, oil of sage, oil of bitter almonds, cassia oil, eucalyptol, citric acid, and fruit essences, including blackcurrant, apple, pear, peach, berry, wildberry, date, blueberry, kiwi, strawberry, raspberry, cherry, plum, pineapple, mango, apricot and citrus oils such as lemon, orange, lime and grapefruit oils, and mixtures thereof.

In a further aspect a method is provided for preparing a pharmaceutical liquid composition comprising an aqueous medium wherein the aqueous medium comprises glycerol and water, wherein the pharmaceutical liquid composition is according to the invention, the method comprising the steps of:
a. providing glycerol;
b. adding the viscosity enhancing agent to the glycerol, while stirring the glycerol to form a mixture of viscosity enhancing agent and glycerol;
c. providing a solution of a sodium salt of benzoic acid and/or the potassium salt of benzoic acid, preferably the sodium salt of benzoic acid, in water;
d. adding the solution of the sodium salt of benzoic acid and/or the potassium salt of benzoic acid to the mixture of viscosity enhancing agent and glycerol, while stirring said mixture to form a solution of the sodium salt of benzoic acid and/or the potassium salt of benzoic acid in the aqueous medium;
and wherein the sodium salt of benzoic acid and/or the potassium salt of benzoic acid has a concentration at least 1.39 mmol/ml in the aqueous medium and wherein the glycerol has a volume concentration of at least 1.0 ml/l relative to the total volume of the aqueous medium.

The viscosity enhancing agent selected comprises a cellulose derivate, the cellulose derivate being selected from the group consisting of methyl-ethyl-propyl cellulose, hydroxypropylcellulose, hydroxy ethyl cellulose, sodium carboxymethyl cellulose, microcrystalline cellulose, wherein preferably the viscosity enhancing agent comprises hydroxyethylcellulose .

In an embodiment, the amount of viscosity enhancing agent added to the glycerol during step b. is selected such that the concentration of the viscosity enhancing agent in the resulting mixture is in the range of 0.01 g/ml to 0.10 g/ml, preferably in the range of 0.02 g/ml to 0.08 g/ml. The amount of viscosity enhancing agent is sufficient to provide the required viscosity of the pharmaceutical liquid composition after step d. adding the solution of the sodium salt of benzoic acid and/or the potassium salt of benzoic acid to the mixture of viscosity enhancing agent and glycerol. The concentration of the viscosity enhancing agent in glycerol is adequate to disperse the viscosity enhancing agent in glycerol. The dispersed viscosity enhancing agent in glycerol may subsequently easily be dissolved by a swelling process in an aqueous medium containing water and glycerol, by adding the solution of the sodium salt of benzoic acid and/or the potassium salt of benzoic acid in water.

In an embodiment, the stirring in step b. is carried out continuously. The stirring process enhances a fine dispersion of the viscosity enhancing agent in glycerol.

The providing of the solution of step c. comprises the steps of:
ii. adding the sodium salt of benzoic acid and/or the potassium salt of benzoic acid in water, while stirring the water.

In an embodiment, further comprising the step of:
e. allowing the aqueous medium to swell, while stirring the aqueous medium.

The step of allowing the aqueous medium to swell according to the present invention is defined as a dissolving process of the dispersed viscosity enhancing agent, while controlling the viscosity of the aqueous medium to be raised, such as raised to a viscosity in the range of 100 mPa.s - 500 mPa.s at 20 °C.

The step of allowing the aqueous medium to swell is carried out after the adding step d.

The time to allow the aqueous medium to swell may be minutes, and may be depending on temperature and stirring speed. In an example, a time of step e. to allow the aqueous medium to swell may in the range 2 - 30 minutes, preferably 5 - 20 minutes.

The stirring of the aqueous medium enhances the swelling process and may shorten the time for the aqueous medium to swell.

In an embodiment, wherein the viscosity enhancing agent is dispersed in glycerol, when adding the solution of the sodium salt of benzoic acid and/or the potassium salt of benzoic acid to the mixture of viscosity enhancing agent and glycerol.

Without restricting the invention it is believed that the dispersed viscosity enhancing agent may have absorbed an amount of glycerol. The dispersion of the viscosity enhancing agent in glycerol enhances an appropriate swelling of the viscosity enhancing agent in an aqueous medium containing water and glycerol to dissolve the viscosity enhancing agent, without disturbing a solution stability of the sodium salt of benzoic acid and/or the potassium salt of benzoic acid, such as sodium benzoate, in the aqueous medium containing water and glycerol.

The method may further comprise the step of:
f. adding a sweetening agent to the solution of a sodium salt of benzoic acid and/or the potassium salt of benzoic acid in water before step d. forming the solution of the sodium salt of benzoic acid and/or the potassium salt of benzoic acid in the aqueous medium.

The addition of the sweetening agent before step d. is advantages to dissolve the sweetening agent in the aqueous medium as the sweetening agent is more easily dissolved in water before allowing the aqueous medium to swell (wherein the viscosity of the aqueous medium is raised to a level higher than a viscosity of water).

In a further aspect a pharmaceutical liquid composition is provided for use as a medicament.

In a further aspect a pharmaceutical liquid composition is provided for use in a method for preventing and/or treating a genetic disorder of a patient.

In an embodiment, the pharmaceutical liquid composition is administered using a nasogastric and/or gastronomy tube. Surprisingly, it is found that the pharmaceutical liquid composition may be reliably administered using a nasogastric and/or gastronomy tube as it is found that the solution of the sodium salt of benzoic acid and/or the potassium salt of benzoic acid remains stable during the use in a nasogastric and/or gastronomy tube.

In a further aspect the kit of parts is provided for use in a method for preventing and/or treating a genetic disorder of a patient.

In an embodiment, the pharmaceutical liquid solution is administered to a patient in need thereof, the administering comprising:
a. adding an amount of the pharmaceutical liquid composition to water to form a mixture, wherein the volume ratio between the pharmaceutical liquid composition and water is in the range of 1:20 to 1:1, preferably in the range of 1:10 to 1:2.
b. Orally, preferably daily, administering said mixture to the patient.

In an embodiment, the pharmaceutical liquid composition is used for daily administering to the patient, preferably in a dose of a therapeutically effective amount of the pharmaceutical liquid composition, more preferably in three or more equally divided liquid doses.

In an embodiment, the amount of the pharmaceutical liquid composition added in step a. to water is in the range 0.5 ml to 20.0 ml.

In an embodiment, the pharmaceutical liquid composition is part of a kit of parts according to the invention, the method further comprising a step before the oral administering step of;
c. adding an amount of said flavouring liquid concentrate, preferably in the form of drops of the at least one flavouring liquid concentrate, to the mixture of step a. or to the water, which is used in step a. to form the mixture.

In particular, the amount of each of said flavouring liquid concentrate added is in the range of 0.01 ml - 1.00 ml, preferably in the range of 0.02 ml - 0.50 ml, more preferably in the range of 0.02 ml - 0.25 ml.

In an embodiment, the kit of parts comprises at least two flavouring liquid concentrates and wherein the step of adding one or more flavouring liquid concentrates comprises selecting an amount of drops of a first flavouring liquid concentrate and selecting an amount of drops of a second flavouring liquid concentrate.

The amount of drops may be in the range of 1 to 10 drops of flavouring liquid concentrate, preferably in the range of 1 to 5 drops of flavouring liquid concentrate. The amount of drops is an accurate way to administer the flavouring liquid concentrate.

### Brief description of the figures

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1 illustrates schematically a process flow for preparing the pharmaceutical liquid composition of an exemplary embodiment;
Figure 2 illustrates schematically individual components of a flavouring pharmaceutical liquid kit.

### Measurement methods

### Viscosity

Viscosity measuring low-viscous Newtonian fluids using Haake viscotester type D. Haake complies to ISO 2555. Measuring temperature is 20 °C, Haake standard spindles are applied (type L1 to L4). Rotational speed: between 0.1 rpm and 200 rpm, measuring viscosity range: between 15 mPas - 2.000.000 mPas.

### Microbiological Stability

According to Assay and Stability methods described in US Pharmacopeia-National Formulary (USP/NF), the European Pharmacopoeia (EP). The antimicrobial effectiveness or preservative effectiveness test is described in the tripartite compendia for sterile parenteral multi-dose formulated products. The procedures used for this test are according to the European Pharmacopeia (Ph. Eur. or EP) 5.1.3 Efficacy of Antimicrobial Preservation.

### Constitution / Concentration Stability

According to Assay and Stability methods described in US Pharmacopeia-National Formulary (USP/NF), the European Pharmacopoeia (EP).

### Taste masking characterization of the pharmaceutical liquid composition

### 1. Phase 1:

During the taste masking process results from taste masking tests are subject to a continuous process evaluation, where progress made is logged by development staff (at least number of test panel persons N=2) on standardized taste perception intensity scale (see below). Subsequently comparing obtained results with initial phase 1 taste profiling results, typical for not-taste masked compositions.

### 2. Phase 2:

First taste masked samples are subsequently further tested using the taste evaluation by a larger group (at least number of test panel persons N=4) and to log taste perception on the standardized perception intensity scale. Logged results are compared and in average validated against logged results of Phase 2.

### Logging protocols flavor perception results

Taste perception results are individually logged by selected taste panel members on standardized taste perception intensity scale, i.e. characterizing perceived bitterness, sourness, sweetness and salty taste, taste perception intensity is graded on a 1-5 scale.

### Taste perception intensity scale for Bitterness:

0 = Not Bitter
1 = Traces, hint of Bitter
2 = Light, underlying Bitterness
3 = Medium, evidently Bitter
4 = Concentrated, strong Bitter
5 = Forceful, overwhelmingly Bitter

Samples in taste panel evaluations are typically packed in blinded packaging's.

### Taste panel members selection/acceptance criteria procedure

Prior to conducting product taste perception testing, taste panel members are screened to verify absence of possibly expected sensitivity/allergic reactions related to the product active or inactive ingredients. Taste panel members with blocked sinuses are excluded from taste evaluations. Subsequently reference products/substances are evaluated on potency and typical intake-levels for panel taste perception testing. Resulting in setting safe for use reference sample intake levels, based on advised single dose quantities/volumes. Taste perception testing is typically performed by means of a "swish & spit" procedure, maximized at 3 tests daily, i.e. at start, middle and end of the business day. Taste evaluation results are individually logged on taste perception protocols by each panel member and interpreted by means of determining a common denominator opinion.

### pH of flavoring concentrates

The flavouring liquid concentrate is dissolved in purified water (e.g. demi water) at a specific concentration of flavouring liquid concentrate in 5 ml purified water. The pH of the purified water itself is determined to be 6.0 - 6.5. The concentration of the concentration of flavouring liquid concentrate is determined by the number of drops added to the purified water multiplied by the weight of the drops. The weight of the drops is about 25 mg per drop of flavouring liquid concentrate. The pH of the flavouring liquid concentrate at the specific concentration is determined at room temperature (in particular at 20 °C).

### Formulation Example

### Pharmaceutical liquid compositions

Pharmaceutical liquid composition based on Sodium benzoate is made according to Example A (200 mg/mL), Example B (300 mg/mL), Example C (400 mg/mL) and Example D (250 mg/mL):

| | | **Example A** | **Example B** | **Example C** | **Example D** |
|---|---|---|---|---|---|
| | **Ingredient** | **Quantity (gram)** | **Quantity (gram)** | **Quantity (gram)** | **Quantity (gram)** |
| 1 | Sodium benzoate | 40.0 | 60.0 | 80.0 | 50.0 |
| 2 | Purified water | 157.4 | 144.8 | 138.0 | 151.1 |
| 3 | Glycerol 100% | 20.0 | 20.0 | 10.0 | 20.0 |
| 4 | Natrosol 250 HX Pharm | 0.8 | 0.8 | 0.4 | 0.8 |
| | Total (1 - 4) | **218.2** | **225.6** | **228.4** | **221.9** |
| | **Sodium benzoate (mg/ml)** | 200 | 300 | 400 | 250 |
| | **pH** | 7.0 | 7.5 - 8.0 | 8.0 | 7.5 |

The Sodium benzoate may be abbreviated as SB in the description.

Natrosol 250 HX Pharm is a Hydroxyethylcellulose and has a weight average M_{w} of 1.000.000 Da.

A concentration of the sodium salt of benzoic acid in the aqueous medium may be determined based on the dry weight of sodium benzoic acid in grams (or milligrams [mg]) with respect to a volume of the aqueous medium in liters (or milliliter [ml]).

A concentration of a sodium salt of benzoic acid and/or the potassium salt of benzoic acid in the Pharmaceutical liquid composition may be determined or assessed by measuring the concentration of benzoic acid in mmol/ml using High-performance liquid chromatography (HPLC) technique. HPLC techniques to determine the concentration of benzoic acid in an aqueous medium are generally known. The HPLC technique may be optimized to separate the benzoic acid from the other components of the pharmaceutical liquid composition depending on the composition of the pharmaceutical liquid composition. Additionally, it may be checked by known techniques that substantially an equal amount of sodium (expressed in mmol/ml) as the benzoic acid (expressed in mmol/ml) is present in the pharmaceutical liquid composition.

### Kit compositions

The kit is comprised of a pharmaceutical liquid composition according to the invention, such as the Pharmaceutical liquid composition A described above, and one or more flavouring liquid concentrate compositions.

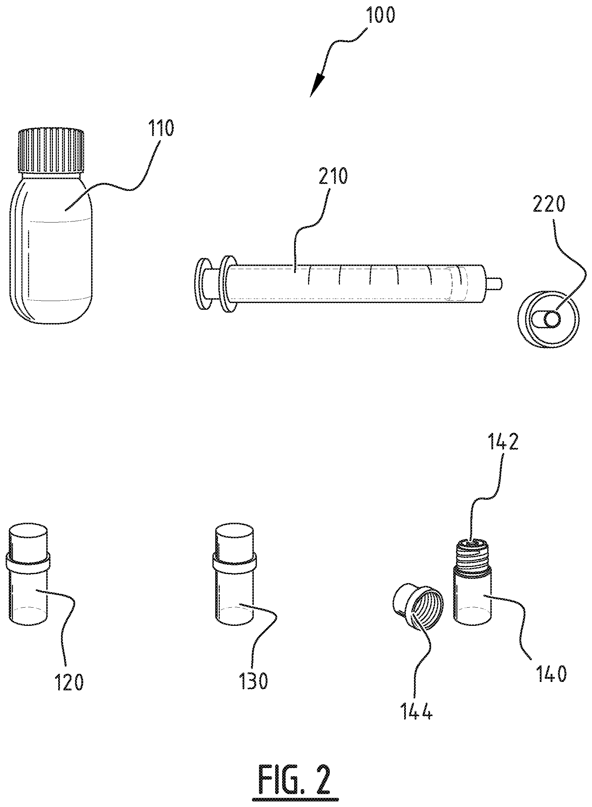

Figure 2 shows a kit 100 including a pharmaceutical liquid composition 110 and three flavouring liquid concentrate compositions 120, 130, 140. The pharmaceutical liquid composition 110 is contained in a bottle, such as a glass bottle. The bottle may contain a volume in the range of 50 ml, 100 ml or 200 ml of the pharmaceutical liquid composition. Optionally a medical syringe 210 may be provided to administer the pharmaceutical liquid composition 110 in an amount of 0.5 ml - 20.0 ml. The medical syringe 210 can be used together with an insert 220, which can be inserted in the bottle 110, to accurately

Preferably, to eliminate common errors in dosing pharmaceutical liquid compositions a specially dosing device is selected, i.e. an oral syringe fitted with a volume dosing (mL) scale and/or a corresponding active substance scale (mg).

Example: a newborn approx. 3.4kg in body weight theoretically requires 4.8mL undiluted of the pharmaceutical composition/day -measured with an accuracy in the range of +/- 5% (at a low volume around 0.5 - 2.5 ml) or even better (at volumes larger than 2.5 ml).

The same oral syringe is fitted with an active substance corresponding weight scale (mg). In this case measuring 4.8mL corresponds with a 1.7 g active substance dose. Where the corresponding active substance weight (mg) correlates with the active substance concentration of the pharmaceutical liquid composition.

Each of the three flavouring liquid concentrate compositions 120, 130, 140 is contained in a smaller bottle, such as a glass bottle. The bottle may contain 3 ml - 10 ml of the flavouring liquid concentrate composition. Each bottle of the flavouring liquid concentrate has a cap 144 and may further contain a medical dropper, such as an dropper insert 142, to administer drops of the flavouring liquid concentrate.

In alternative embodiments, the kit 100 may have one flavouring liquid concentrate composition contained in a bottle, may have two flavouring liquid concentrate compositions individually packed in a bottle, or any other suitable number of flavouring liquid concentrate composition individually packed in a bottle.

### Flavouring liquid concentrate compositions

### Batch formula; - Mango-Orange drops - inactive ingredient - topping flavor composition A

| | **Ingredient** | **Quantity (p/gram)** | **Quantity (g) p/100mL batch (100mL = ± 92g)** |
|---|---|---|---|
| 1 | Mango CRA 1221L | 0.495 | 45.54 |
| 2 | Orange CRA 1028L | 0.388 | 35.81 |
| 3 | Peppermint CRA 1418L | 0.117 | 10.79 |
| | | 1.000 | 92.33 |

### Batch formula; - Blackcurrant drops - inactive ingredient - topping flavor composition B

| | **Ingredient** | **Quantity (p/gram)** | **Quantity (g) p/100mL batch (100mL = ± 96g)** |
|---|---|---|---|
| 1 | Blackcurrant CRA 1198L | 0.885 | 84.96 |
| 2 | Peppermint CRA 1418L | 0.115 | 11.04 |
| | | 1.000 | 96.00 |

### Batch formula; - Lemon-Mint drops - inactive ingredient - topping flavor composition C

| | **Ingredient** | **Quantity (p/gram)** | **Quantity (g) p/100mL batch (100mL = ± 87g)** |
|---|---|---|---|
| 1 | Lemon CRA 1190L | 0.794 | 69.44 |
| 2 | Peppermint CRA 1418L | 0.206 | 18.06 |
| | | 1.000 | 87.50 |

### Methods for preparing the compositions

### Pharmaceutical liquid composition

### Equipment: stainless steel tanks (2), propeller stirrer (preferably portable), bottles filling line

### Description of the manufacturing process:

1. Dispense raw materials
2. Fill a suitable primary stainless steel tank with the Glycerol 100%
3. Gradually add and disperse the Natrosol 250 HX Pharm in the Glycerol, whilst stirring continuously
4. Verify visually if the Natrosol 250 HX Pharm is homogeneously dispersed
5. Fill a suitable secondary stainless steel tank with the available purified water
6. Gradually (step-by-step) add and dissolve the Sodium Benzoate in the Purified water, whilst stirring continuously
7. Verify visually if the Sodium Benzoate is completely dissolved
8. Gradually add the Sodium Benzoate solution to the primary tank with the Glycerol 100%/Natrosol 250 HX Pharm dispersion, whilst stirring continuously
9. Allow the complete solution to swell for several minutes (Example: 10-15 min at 2 liter scale), whilst stirring continuously
10. Verify visually if the final solution is homogeneous and clear
11. Sample the bulk solution for in-process analysis prior to filling, verifying assay Sodium Benzoate
12. Fill the bulk solution into selected bottles
13. Sample filled bottles for final product QC release analysis according to product specifications
14. Label the bottles

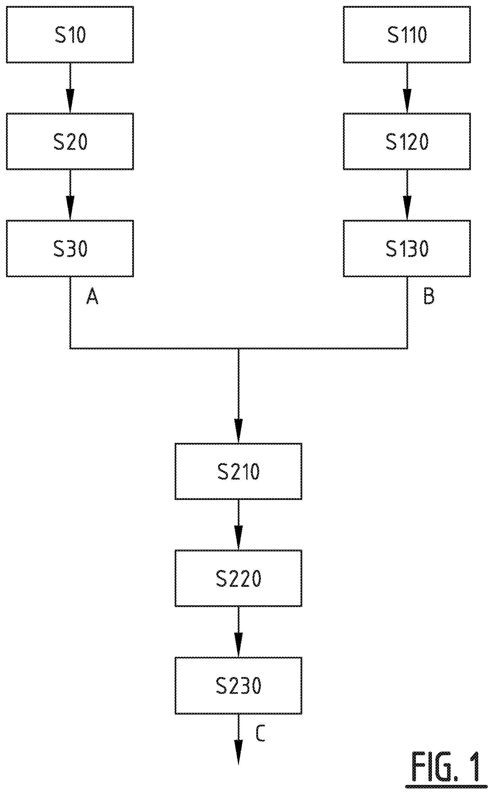

A workflow for a process of preparing the pharmaceutical liquid composition according to an embodiment is shown in Fig. 1.

In step S10 a first tank is provided with glycerol. In step S20 a viscosity enhancing agent, preferably a cellulose derivate, in an example Natrosol 250 HX, is gradually added and dispersed in the glycerol, while stirring the glycerol continuously.

In step S30 it is verified, such as visually, that the viscosity enhancing agent is homogeneously dispersed in the glycerol. After step S30 a mixture A of viscosity enhancing agent and glycerol is provided, wherein the viscosity enhancing agent is dispersed in glycerol.

In step S110 a second tank is provided with purified water. In step S120 a physiologically acceptable salt of benzoic acid, such as sodium benzoate, is gradually added and dissolved in the water, while stirring the solution continuously.

In step S130 it is verified, such as visually, that the salt of benzoic acid is dissolved in water. After step S130 a solution B of salt of benzoic acid is provided.

In step S210 the solution B is gradually added to the mixture A of viscosity enhancing agent and glycerol, while stirring the mixture continuously. After adding the solution B, in step S220 the aqueous medium of the mixture is allowed to swell for minutes, while stirring the aqueous medium continuously. In step S230 it is verified, such as visually, that the final solution C is homogeneous and clear. The viscosity enhancing agent is completely dissolved in the aqueous medium.

In an example, the solution B has a concentration of sodium benzoate such that the concentration of sodium benzoate in the solution C is at least 250 mg/ml.

### Flavouring liquid concentrate compositions

### Manufacturing method;

Equipment: Erlenmeyer flask with cover, portable lab-scale propeller stirrer, bottles filling equipment. Note: possibility to flush Erlenmeyer flask and bottles with Nitrogen (prior/during mixing/filling)

### Description of the manufacturing process:

1. Dispense raw materials
2. Flush the Erlenmeyer flask with nitrogen prior to adding ingredients and close the flask
3. Open the flask briefly and add the liquid flavoring agents, flush the tank again with nitrogen
4. Close the flask directly after and mix ingredients for approx. 10 minutes
5. Verify visually if the blend is homogeneous, sample the mixture for release
6. Flush the flask again with nitrogen and close the flask
7. Fill the bulk mixture into selected bottles, whilst flushing with nitrogen
8. Sample filled bottles for final product QC release analysis according to product specifications
9. Label the bottle

### Properties

### Solution stability

Testing of solubility of Sodium Benzoate (SB) in purified water at concentrations of 200 mg/ml, 300 mg/ml, 400 mg/ml and 250mg/ml demonstrated that the latter concentration was the most efficient concentration for implementation in a conventional manufacturing process.

Solutions according to Example A - Example D were packed in clear glass bottles and stored for 1.5 month at 25°C, and the Example C (400 mg/ml) was also stored at 40°C. None of the Examples A - D showed crystallization at 25°C. Even Example C showed no showed crystallization at 40°C.

Considering the technical difficulties associated with dissolving SB at concentrations higher than 400 mg/ml, it was concluded that the risk of creating a near saturated SB solution above the 400 mg/ml concentration was present.

This selection is particularly relevant because the previous represents a baseline formulation to which a number of additional (taste masking) supportive inactive ingredients required to completely dissolve in the available aqueous component of the formulation.

### Preservation Effect of Pharmaceutical liquid composition

Conducted Preservative Efficacy Tests (PET) and Bioburden tests with formulations, containing SB 250 mg/mL (Example D) unexpectedly confirmed self-preservation properties of SB in aqueous solutions. Unexpected results because in theory any preservation effect of SB at the alkaline pH of the solution would not be expected.

Challenge tests have been carried out with the formulation of Example D. The formulation of Example D passed the Challenge tests for oral preparations according to method EP.5.1.3..

Supportive bacteriostatic, fungicidal properties of glycerol were considered to substantiate self-preservation properties of SB 250 mg/mL in aqueous solutions.

The Example D, however, only has a concentration of about 10 weight-% of glycerol, which on its own does not explain the self-preservation properties of SB 250 mg/mL.

Thus, the results show that the concentration level of the sodium salt of benzoic acid and/or the potassium salt of benzoic acid of at least 1.39 mmol/ml contribute to a high microbiological stability of the pharmaceutical liquid composition.

### Viscosity

The measurement of viscosity gave the following results:
Dynamic viscosity of water is 1.00 mPa-s at 20°C.
Dynamic viscosity of glycerol is 1.31mPa-s at 20°C.
Dynamic viscosity of glycerol-water mixture (at 10 % w/v) is 1.36 mPa-s at 20°C.
Dynamic viscosity of Pharmaceutical liquid compositions D is about 250 mPa-s at 20°C.

### Flavouring liquid concentrate drops

The Flavouring liquid concentrates may be dosed in drops by a medical dropper in drop volumes corresponding to 20 - 40 drops per mL. The drops have a weight in the range of 15mg to 50mg per drop. The average weight of the drops during the test with the flavouring liquid concentrates is about 25 mg per drop.

### Taste masking test

### Phase 1; Sodium benzoate 200mg, 250mg, 300mg and 400mg/mL aqueous solutions evaluation

Manufactured Sodium benzoate solutions were evaluated by three taste panel members (N=3) as follows;
- Safe for 'swish & spit" taste evaluation product dose levels were established and participating taste panel members were screened according to set selection, acceptance criteria procedures as described above;
- Each Sodium benzoate solution was sampled in eight equal volumes (7mL) for each panel member, blinded and randomly numbered;
- Seven Sodium benzoate solution taste evaluation samples were 1:5 diluted with purified water and one sample remained undiluted;
- Subsequently, six 1:5 diluted samples were evaluated with respectively 1 & 3 drops of developed Mango-Orange, Blackcurrant, Lemon-Mint flavoring drops compositions (topping flavor composition A - C). And one sample was evaluated as a 1:5 diluted with purified water without additives;
- Each panel member logged taste perception for each sample on standardized taste perception intensity scale protocols.
- Additional (1): the acidity/alkalinity (pH) of each solution was investigated and logged
- Additional (2): extra Sodium benzoate 250mg/mL solution samples were manufactured and evaluated, (1:5 & 1:10) diluted with commercially available Lemonade syrup and Orange juice

### Results Phase1, taste perception evaluation;

See the results of the Phase 1-taste evaluation average scores (bitterness) in the following table P1:

**Table P1: taste evaluation average scores (bitterness) Phase 1**

| **Condition** | **Example A** | **Example B** | **Example C** | **Example D** |
|---|---|---|---|---|
| Undiluted | 5 | 5 | 5 | 5 |
| 1:5 water diluted | 3 | 3 | 4 | 3 |
| 1:5 water diluted with 3 drops of flavor composition C | 2 | 3 | 3 | 2 |
| 1:5 water diluted with 3 drops of flavor composition A | 2 | 2 | 3 | 2 |
| 1:5 water diluted Sodium benzoate solutions with 3 drops flavor composition B | 1 | 2 | 2 | 1 |

The taste of undiluted Sodium benzoate solutions in all concentrations (A - D) were in average negatively perceived as overwhelmingly bitter (5), confirming necessity of the taste masking process. Respective pH values were; 200mg/mL-pH 7, 250mg/mL-pH 7.5, 300mg/mL-pH 7.5-8 and 400mg/mL-pH 8. The purified water has a pH value of 6.0 - 6.5.

Taste perception of 1:5 diluted Sodium benzoate solution samples without added flavor composition drops at 400mg/mL concentration remained strong bitter (4) but predominantly in the aftertaste. The 200mg/mL and 250mg/mL and 300mg/mL concentrations, which were 1:5 diluted, were in average disliked and were graded as evidently bitter (3).

Respective pH values were; 200mg/mL-pH 6.5-7, 250mg/mL-pH 7, 300mg/mL-pH 7-7.5 and 400mg/mL-pH 7.5-8.

The dilution of Sodium benzoate solution samples with the purified water slightly lowers the pH in a range (0 - 0.5) with respect to non-diluted Sodium benzoate solutions.

The drops of the flavor compositions used had a weight of about 25 mg per drop.

Taste perception of concentrations 1:5 diluted Sodium benzoate solution A and D samples with 3 added drops of flavor composition A and C were in average graded as having light underlying bitterness (2). With a clear preference for the added Blackcurrant flavor drops composition version, having traces or hint of bitterness (1).

The 400mg/mL concentration was still perceived as medium, evidently bitter (3) in the aftertaste with 3 drops of each of the flavor compositions A and C, respectively, likely due to the relatively high Sodium benzoate concentration. Also for the 400mg/mL concentration, the flavor composition B improved the taste the best of the flavor compositions ( 2 instead of 3)

The pH effect of the three flavor compositions A - C was compared to one another using the pH test described above, by dissolving a specific amount of drops of flavor composition in purified water. The pH of the purified water is 6.0 - 6.5.

pH values of solutions with 1 added drop, 3 added drops and 10 added drops in 5 ml purified water were tested for each of the flavor compositions A - C:

| | pH | pH | pH |
|---|---|---|---|
| **Number of Drops** | **1** | **3** | **10** |
| Composition A | 5.5 | 4.5 | 3.5 |
| Composition B | 6.0 | 6.5 | 6.5-7.0 |
| Composition C | 5.0 | 4.5 | 4.0 |

pH values of solutions with 1 added drop, 3 added drops and 10 added drops of the Blackcurrant composition B was found to be 6.0 - 6.5. The pH of 1:5 diluted Sodium benzoate solutions with 1 drop (about 25 mg) Lemon-Mint C and Mango-Orange A compositions were found to be more acidic (pH 5.0 -5.5). When adding more drops, 3 drops (about 75 mg in total) and 10 drops (about 250 mg in total), for Lemon-Mint C and Mango-Orange A compositions the acidity further increased to pH 4.5 (for 3 drops) or to pH 4.0 or lower (for 10 drops).

Based on these results, it is shown that the pH using the Blackcurrant composition B remains above 6.5, even when using a higher amount (concentration) of flavor composition. This explains the more consistent and better taste masking results for the Blackcurrant composition B for the Sodium benzoate solutions.

Additional taste evaluations with Sodium benzoate 250mg/mL solutions, 1:5 & 1:10 diluted with commercially available Lemonade syrup (LS) and Orange juice (OJ) were graded as not palatable and unacceptable (5: Forceful, overwhelmingly Bitter). The result is much worse compared to a 1:5 dilution using purified water. The respective pH level decreased to 3-4 with increasing LS and OJ concentrations, confirming a linked increasing deterioration of the product flavor link to an increasing acidity (pH <6) of Sodium Benzoate solutions.

Based taste preference scores and physical/chemical considerations with the various tested Sodium benzoate concentrations a 250mg/mL concentration was selected for Phase23 taste evaluations with respectively 1 & 3 drops of developed Mango-Orange, Blackcurrant, Lemon-Mint flavoring drops compositions A - C.

### Phase 2; Sodium benzoate 250mg/mL aqueous solutions evaluation

Manufactured Sodium benzoate solutions were evaluated at the by five test panel members (N=5) as follows;
- Safe for 'swish & spit" taste evaluation product dose levels were established and participating taste panel members were screened according to set selection, acceptance criteria procedures
- The Sodium benzoate 250mg/mL solution was sampled in equal volumes (7mL) for each panel member, blinded and randomly numbered
- Sodium benzoate solution taste evaluation samples were 1:5 diluted with purified water and one sample remained undiluted
- Subsequently, 1:5 diluted samples were evaluated with respectively 1 & 3 drops of Mango-Orange, Blackcurrant, Lemon-Mint flavoring drops compositions (topping flavor composition A - C).
- Each panel member logged taste perception for each sample on standardized taste perception intensity scale protocols.

### Results Phase 2, taste perception evaluation;

See the results of the Phase 2-taste evaluation average scores (bitterness) in the following table P2:

**Table P2: taste evaluation average scores (bitterness) Phase 2**

| **Condition** | **Example D** |
|---|---|
| Undiluted | 5 |
| 1:5 water diluted | 3 |
| 1:5 water diluted with 3 drops of flavor composition C | 2 |
| 1:5 water diluted with 3 drops of flavor composition A | 1 - 2 |
| 1:5 water diluted Sodium benzoate solutions with 3 drops flavor composition B | 1 |

The taste of undiluted Sodium benzoate 250mg/mL solution in average was negatively perceived as Forceful, overwhelmingly Bitter (5), reconfirming necessity of the taste masking process.

Taste perception of the 1:5 diluted Sodium benzoate solution samples with 3 added drops of each flavor composition (A - C), respectively, was in average graded as Light, underlying Bitterness (2). Blackcurrant flavor drops were in average graded as a first preference (1) and the added Mango-Orange drops version as second best (1 - 2). Reconfirming Phase 2 taste evaluation results and the link between taste deterioration and an increasing acidity of Sodium Benzoate solutions.

It should be appreciated by those skilled in the art that any block diagrams herein represent conceptual views of illustrative units or modules embodying the principles of the invention.

Whilst the principles of the invention have been set out above in connection with specific embodiments, it is to be understood that this description is merely made by way of example and not as a limitation of the scope of protection which is determined by the appended claims.

## Claims

1. A pharmaceutical liquid composition comprising a solution of the sodium salt of benzoic acid and/or the potassium salt of benzoic acid, in an aqueous medium at a concentration of at least 1.39 mmol/ml;
wherein the aqueous medium comprises:
a) glycerol having a volume concentration of at least 1.0 ml/l relative to the total volume of the aqueous medium;
b) a viscosity enhancing agent comprising a cellulose derivate being selected from the group consisting of methyl-ethyl-propyl cellulose, hydroxypropylcellulose, hydroxyethylcellulose, sodium carboxymethyl cellulose, microcrystalline cellulose; and
c) water;
and wherein the pH of the liquid composition is in the range between 6.0 and 10.0, and wherein the weight ratio between the glycerol and the viscosity enhancing agent is between 200 : 1 and 10 : 1.

2. Pharmaceutical liquid composition according to claim 1, wherein the viscosity enhancing agent:
has a concentration of at least 0.001 g/ml relative to the total volume of the aqueous medium, preferably in the range 0.001 g/ml to 1.000 g/ml relative to the total volume of the aqueous medium, more preferably in the range 0.001 g/ml to 0.100 g/ml relative to the total volume of the aqueous medium.

3. Pharmaceutical liquid composition according any one of the preceding claims, wherein:
the viscosity of the liquid composition is in the range of 100 mPa.s - 500 mPa.s at 20 °C; and/or
the viscosity enhancing agent comprises a hydroxyethylcellulose having a weight average molecular weight in the range of 200.000 - 2.000.000 Da.

4. A kit of parts comprising:
a. a pharmaceutical liquid composition according to any one of claims 1- 3; and
b. a flavouring liquid concentrate, the flavouring concentrate comprising at least one flavouring agent, preferably a first flavouring agent and a second flavouring agent.

5. The kit of parts according to claim 4, wherein the first flavouring agent is selected from the group of mint, ginger, caramel, liquorice, peppermint and eucalyptol and mixtures thereof, wherein preferably the second flavouring agent is a fruit essence.

6. The kit of parts according to claim 4 or 5, wherein the flavouring liquid concentrate, when dissolved in purified water at a concentration of 0.10 g / 5 ml water, has a pH of at least 5.5, and preferably, when dissolved in purified water at a concentration of a concentration of 0.25 g / 5 ml water, has a pH of at least 6.0.

7. The kit of parts according to any one of the claims 4-6, wherein:
each flavouring agent has a concentration in the range 2- 100 weight-% of the total weight of the flavouring liquid concentrate and the sum of the at least one flavouring agent of the flavouring liquid concentrate is in the range of 80 - 100 weight-% of the total weight of the flavouring liquid concentrate; and/or
the kit comprises a medical dropper for administering drops of the at least one flavouring liquid concentrate.

8. A method for preparing a pharmaceutical liquid composition comprising an aqueous medium wherein the aqueous medium comprises glycerol and water, wherein the pharmaceutical liquid composition is according to claim 1, the method comprising the steps of:
a. providing glycerol;
b. adding the viscosity enhancing agent to the glycerol, while stirring, preferably continuously, the glycerol to form a mixture of viscosity enhancing agent and glycerol, wherein the viscosity enhancing agent comprises a cellulose derivate selected from the group consisting of methyl-ethyl-propyl cellulose, hydroxypropylcellulose, hydroxy ethyl cellulose, sodium carboxymethyl cellulose, microcrystalline cellulose;
c. providing a solution of the sodium salt of benzoic acid or the potassium salt of benzoic acid, in water;
d. adding the solution of the salt of benzoic acid to the mixture of viscosity enhancing agent and glycerol, while stirring said mixture to form a solution of the physiologically acceptable salt of benzoic acid in the aqueous medium;
e. optionally allowing the aqueous medium to swell, while stirring the aqueous medium;
wherein the physiologically acceptable salt of benzoic acid has a concentration of at least 1.39 mmol/ml in the aqueous medium, and wherein the glycerol has a volume concentration of at least 1.0 ml/l relative to the total volume of the aqueous medium.

9. Method for preparing the pharmaceutical liquid composition according to claim 8, wherein:
the amount of viscosity enhancing agent added to the glycerol during step b. is selected such that the concentration of the viscosity enhancing agent in the resulting mixture is in the range of 0.01 g/ml to 0.10 g/ml, preferably in the range of 0.02 g/ml to 0.08/ml; and/or
the viscosity enhancing agent is dispersed in glycerol when adding the solution of the physiologically acceptable salt of benzoic acid to the mixture of viscosity enhancing agent and glycerol.

10. Pharmaceutical liquid composition according to claim 1 or kit of parts according to claim 5 for use as a medicament, preferably for preventing and/or treating a genetic disorder of a patient.

11. Pharmaceutical liquid composition for use according to claim 10, wherein the pharmaceutical liquid composition is administered using a nasogastric and/or gastronomy tube.

12. Pharmaceutical liquid composition for use according to claim 10, wherein the pharmaceutical liquid solution is administered to a patient in need thereof, the administering comprising:
a. adding an amount of the pharmaceutical liquid composition to water to form a mixture, wherein the volume ratio between the pharmaceutical liquid composition and water is in the range of 1:20 to 1:1, preferably in the range of 1:10 to 1:2.
b. orally, preferably daily, administering said mixture to the patient; optionally wherein the amount of the pharmaceutical liquid composition added in step a. to water is in the range 0.5 ml to 20.0 ml.

13. Pharmaceutical liquid composition for use according to claim 10, 11 or 12, wherein the pharmaceutical liquid composition is used for daily administering to the patient, preferably in a dose of a therapeutically effective amount of the pharmaceutical liquid composition, more preferably in three or more equally divided liquid doses.

14. Pharmaceutical liquid formulation for use according to claim 12, wherein the pharmaceutical liquid composition is part of a kit of parts according to claim 4, the method further comprising a step before the oral administering step of;
c. adding an amount of said flavouring liquid concentrate, preferably in the range of 0.01 ml - 1.00 ml, preferably in the range of 0.02 ml - 0.50 ml, more preferably in the range of 0.02 ml - 0.25 ml, to the mixture of step a. or to the water, which is used in step a. to form the mixture;
optionally wherein the kit of parts comprises at least two flavouring liquid concentrates and wherein the step of adding one or more flavouring liquid concentrates comprises selecting an amount of drops of a first flavouring liquid concentrate and selecting an amount of drops of a second flavouring liquid concentrate.

## Patentansprüche

1. Pharmazeutische flüssige Zusammensetzung, umfassend eine Lösung des Natriumsalzes von Benzoesäure und/oder des Kaliumsalzes von Benzoesäure in einem wässrigen Medium bei einer Konzentration von mindestens 1,39 mmol/ml;
wobei das wässrige Medium umfasst:
a) Glycerin, das eine Volumenkonzentration von mindestens 1,0 ml/l bezüglich des Gesamtvolumens des wässrigen Mediums aufweist;
b) ein viskositätserhöhendes Mittel, umfassend ein Cellulosederivat, das ausgewählt ist aus der Gruppe, bestehend aus Methyl-Ethyl-Propyl-Cellulose, Hydroxypropylcellulose, Hydroxyethylcellulose, Natriumcarboxymethylcellulose, mikrokristalliner Cellulose; und
c) Wasser;
und wobei der pH-Wert der flüssigen Zusammensetzung in dem Bereich zwischen 6,0 und 10,0 liegt, und wobei das Gewichtsverhältnis zwischen dem Glycerin und dem viskositätserhöhenden Mittel zwischen 200 : 1 und 10 : 1 liegt.

2. Pharmazeutische flüssige Zusammensetzung nach Anspruch 1, wobei das viskositätserhöhende Mittel:
eine Konzentration von mindestens 0,001 g/ml bezüglich des Gesamtvolumens des wässrigen Mediums, vorzugsweise in dem Bereich 0,001 g/ml bis 1.000 g/ml bezüglich des Gesamtvolumens des wässrigen Mediums, mehr bevorzugt in dem Bereich 0,001 g/ml bis 0,100 g/ml bezüglich des Gesamtvolumens des wässrigen Mediums, aufweist.

3. Pharmazeutische flüssige Zusammensetzung nach einem der vorstehenden Ansprüche, wobei:
die Viskosität der flüssigen Zusammensetzung in dem Bereich von 100 mPa.s-500 mPa.s bei 20 °C liegt; und/oder
das viskositätserhöhende Mittel eine Hydroxyethylcellulose umfasst, die ein gewichtsmittleres Molekulargewicht in dem Bereich von 200.000-2.000.000 Da aufweist.

4. Kit von Teilen, umfassend:
a. eine pharmazeutische flüssige Zusammensetzung nach einem der Ansprüche 1 bis 3; und
b. ein flüssiges Aromatisierungskonzentrat, das Aromatisierungskonzentrat umfassend mindestens ein Aromatisierungsmittel, vorzugsweise ein erstes Aromatisierungsmittel und ein zweites Aromatisierungsmittel.

5. Kit von Teilen nach Anspruch 4, wobei das erste Aromatisierungsmittel ausgewählt ist aus der Gruppe von Minze, Ingwer, Karamell, Lakritze, Pfefferminze und Eukalyptol und Mischungen davon, wobei vorzugsweise das zweite Aromatisierungsmittel eine Fruchtessenz ist.

6. Kit von Teilen nach Anspruch 4 oder 5, wobei das flüssige Aromatisierungskonzentrat, wenn es in gereinigtem Wasser bei einer Konzentration von 0,10 g / 5 ml Wasser gelöst wird, einen pH-Wert von mindestens 5,5 aufweist, und vorzugsweise, wenn es in gereinigtem Wasser bei einer Konzentration von 0,25 g / 5 ml Wasser gelöst wird, einen pH-Wert von mindestens 6,0 aufweist.

7. Kit von Teilen nach einem der Ansprüche 4 bis 6, wobei:
jedes Aromatisierungsmittel eine Konzentration in dem Bereich von 2-100 Gew.-% des Gesamtgewichts des flüssigen Aromatisierungskonzentrats aufweist und die Summe des mindestens einen Aromatisierungsmittels des Aromatisierungskonzentrats in dem Bereich von 80-100 Gew.-% des Gesamtgewichts des flüssigen Aromatisierungskonzentrats liegt; und/oder
das Kit einen medizinischen Tropfer zum Verabreichen von Tropfen des mindestens einen flüssigen Aromatisierungskonzentrats umfasst.

8. Verfahren zum Herstellen einer pharmazeutischen flüssigen Zusammensetzung, umfassend ein wässriges Medium, wobei das wässrige Medium Glycerin und Wasser umfasst, wobei die pharmazeutische flüssige Zusammensetzung nach Anspruch 1 vorliegt, das Verfahren umfassend die Schritte:
a. Bereitstellen von Glycerin;
b. Hinzufügen des viskositätserhöhenden Mittels zu dem Glycerin unter Rühren, vorzugsweise kontinuierlich, des Glycerins, um eine Mischung aus viskositätserhöhendem Mittel und Glycerin auszubilden, wobei das viskositätserhöhende Mittel ein Cellulosederivat umfasst, ausgewählt aus der Gruppe, bestehend aus Methyl-Ethyl-Propyl-Cellulose, Hydroxypropylcellulose, Hydroxyethylcellulose, Natriumcarboxymethylcellulose, mikrokristalliner Cellulose;
c. Bereitstellen einer Lösung des Natriumsalzes von Benzoesäure oder des Kaliumsalzes von Benzoesäure in Wasser;
d. Hinzufügen der Lösung des Salzes von Benzoesäure zu der Mischung aus viskositätserhöhendem Mittel und Glycerin unter Rühren der Mischung, um eine Lösung des physiologisch verträglichen Salzes von Benzoesäure in dem wässrigen Medium auszubilden;
e. optional Quellenlassen des wässrigen Mediums unter Rühren des wässrigen Mediums;
wobei das physiologisch verträgliche Salz von Benzoesäure eine Konzentration von mindestens 1,39 mmol/ml in dem wässrigen Medium aufweist, und wobei das Glycerin eine Volumenkonzentration von mindestens 1,0 ml/1 bezüglich des Gesamtvolumens des wässrigen Mediums aufweist.

9. Verfahren zum Herstellen der pharmazeutischen flüssigen Zusammensetzung nach Anspruch 8, wobei:
die Menge von viskositätserhöhendem Mittel, das dem Glycerin während Schritt b. hinzugefügt wird, derart ausgewählt ist, dass die Konzentration des viskositätserhöhenden Mittels in der resultierenden Mischung in dem Bereich von 0,01 g/ml bis 0,10 g/ml, vorzugsweise in dem Bereich von 0,02 g/ml bis 0,08 g/ml, liegt; und/oder
das viskositätserhöhende Mittel in Glycerin dispergiert wird, wenn die Lösung des physiologisch verträglichen Salzes von Benzoesäure zu der Mischung aus viskositätserhöhendem Mittel und Glycerin hinzugefügt wird.

10. Pharmazeutische flüssige Zusammensetzung nach Anspruch 1 oder Kit von Teilen nach Anspruch 5 zur Verwendung als ein Arzneimittel, vorzugsweise zum Vorbeugen und/oder Behandeln einer genetischen Erkrankung eines Patienten.

11. Pharmazeutische flüssige Zusammensetzung zur Verwendung nach Anspruch 10, wobei die pharmazeutische flüssige Zusammensetzung unter Verwendung einer nasogastralen und/oder Gastronomiesonde verabreicht wird.

12. Pharmazeutische flüssige Zusammensetzung zur Verwendung nach Anspruch 10, wobei die pharmazeutische flüssige Lösung einem Patienten, der diese benötigt, verabreicht wird, das Verabreichen umfassend:
a. Hinzufügen einer Menge der pharmazeutischen flüssigen Zusammensetzung zu Wasser, um eine Mischung auszubilden, wobei das Volumenverhältnis zwischen der pharmazeutischen flüssigen Zusammensetzung und Wasser in dem Bereich von 1 : 20 bis 1 : 1, vorzugsweise in dem Bereich von 1 : 10 bis 1 : 2, liegt.
b. orales, vorzugsweise tägliches, Verabreichen der Mischung an den Patienten;
optional wobei die Menge der pharmazeutischen flüssigen Zusammensetzung, die in Schritt a. zu Wasser hinzugefügt wird, in dem Bereich von 0,5 ml bis 20,0 ml liegt.

13. Pharmazeutische flüssige Zusammensetzung zur Verwendung nach einem der Ansprüche 10, 11 oder 12, wobei die pharmazeutische flüssige Zusammensetzung zum täglichen Verabreichen an den Patienten verwendet wird, vorzugsweise in einer Dosis einer therapeutisch wirksamen Menge der pharmazeutischen flüssigen Zusammensetzung, mehr bevorzugt in drei oder mehr gleichmäßig aufgeteilten flüssigen Dosen.

14. Pharmazeutische flüssige Zusammensetzung zur Verwendung nach Anspruch 12, wobei die pharmazeutische flüssige Zusammensetzung Teil eines Kits von Teilen nach Anspruch 4 ist, das Verfahren ferner umfassend einen Schritt vor dem Schritt des oralen Verabreichens;
c. Hinzufügen einer Menge des flüssigen Aromatisierungskonzentrats, vorzugsweise in dem Bereich von 0,01 ml-1,00 ml, vorzugsweise in dem Bereich von 0,02 ml-0,50 ml, mehr bevorzugt in dem Bereich von 0,02 ml-0,25 ml, zu der Mischung aus Schritt a. oder zu dem Wasser, das in Schritt a. verwendet wird, um die Mischung auszubilden;
optional wobei das Kit von Teilen mindestens zwei flüssige Aromatisierungskonzentrate umfasst und wobei der Schritt des Hinzufügens eines oder mehrerer flüssiger Aromatisierungskonzentrate das Auswählen einer Menge von Tropfen eines ersten flüssigen Aromatisierungskonzentrats und das Auswählen einer Menge von Tropfen eines zweiten flüssigen Aromatisierungskonzentrats umfasst.

## Revendications

1. Composition liquide pharmaceutique comprenant une solution du sel de sodium d'acide benzoïque et/ou du sel de potassium d'acide benzoïque, dans un milieu aqueux à une concentration d'au moins 1,39 mmol/ml ;
dans laquelle le milieu aqueux comprend :
a) du glycérol ayant une concentration volumique d'au moins 1,0 ml/l par rapport au volume total du milieu aqueux ;
b) un agent augmentant la viscosité comprenant un dérivé de cellulose choisi dans le groupe constitué de méthyl-éthyl-propylcellulose, hydroxypropylcellulose, hydroxyéthylcellulose, carboxyméthylcellulose sodique, cellulose microcristalline ; et
c) de l'eau ;
et dans laquelle le pH de la composition liquide est compris dans la plage entre 6,0 et 10,0, et dans laquelle le rapport en poids entre le glycérol et l'agent augmentant la viscosité est compris entre 200: 1 et 10:1.

2. Composition liquide pharmaceutique selon la revendication 1, dans lequel l'agent augmentant la viscosité :
a une concentration d'au moins 0,001 g/ml par rapport au volume total du milieu aqueux, comprise de préférence dans la plage de 0,001 g/ml à 1 000 g/ml par rapport au volume total du milieu aqueux, plus préférablement dans la plage de 0,001 g/ml à 0,100 g/ml par rapport au volume total du milieu aqueux.

3. Composition liquide pharmaceutique selon l'une quelconque des revendications précédentes, dans laquelle :
la viscosité de la composition liquide est comprise dans la plage de 100 mPa.s à 500 mPa.s à 20 °C ; et/ou
l'agent augmentant la viscosité comprend une hydroxyéthylcellulose ayant une masse moléculaire moyenne en poids comprise dans la plage de 200 000 à 2 000 000 Da.

4. Trousse de parties comprenant :
a. une composition liquide pharmaceutique selon l'une quelconque des revendications 1 à 3 ; et
b. un concentré liquide aromatisant, le concentré aromatisant comprenant au moins un agent aromatisant, de préférence un premier agent aromatisant et un second agent aromatisant.

5. Trousse de parties selon la revendication 4, dans laquelle le premier agent aromatisant est choisi dans le groupe de menthe, gingembre, caramel, réglisse, menthe poivrée et eucalyptol et mélanges de ceux-ci, dans laquelle le second agent aromatisant est de préférence une essence de fruit.

6. Trousse de parties selon la revendication 4 ou 5, dans lequel le concentré liquide aromatisant, lorsqu'il est dissous dans de l'eau purifiée à une concentration de 0,10 g/5 ml d'eau, a un pH d'au moins 5,5, et de préférence, lorsqu'il est dissous dans de l'eau purifiée à une concentration d'une concentration de 0,25 g/5 ml d'eau, a un pH d'au moins 6,0.

7. Trousse de parties selon l'une quelconque des revendications 4 à 6, dans laquelle :
chaque agent aromatisant a une concentration comprise dans la plage de 2 à 100 % en poids du poids total du concentré liquide aromatisant et la somme de l'au moins un agent aromatisant du concentré liquide aromatisant est comprise dans la plage de 80 à 100 % en poids du poids total du concentré liquide aromatisant ; et/ou
la trousse comprend un compte-gouttes médical permettant d'administrer des gouttes de l'au moins un concentré liquide aromatisant.

8. Procédé permettant de préparer une composition liquide pharmaceutique comprenant un milieu aqueux, dans lequel le milieu aqueux comprend du glycérol et de l'eau, dans lequel la composition liquide pharmaceutique est selon la revendication 1, le procédé comprenant les étapes consistant à :
a. fournir du glycérol ;
b. ajouter l'agent augmentant la viscosité au glycérol, tout en agitant, de préférence en continu, le glycérol pour former un mélange d'agent augmentant la viscosité et de glycérol, dans lequel l'agent augmentant la viscosité comprend un dérivé de cellulose choisi dans le groupe constitué de méthyl-éthyl-propylcellulose, hydroxypropylcellulose, hydroxyéthylcellulose, carboxyméthylcellulose sodique, cellulose microcristalline ;
c. fournir une solution du sel de sodium d'acide benzoïque ou du sel de potassium d'acide benzoïque, dans de l'eau ;
d. ajouter la solution du sel d'acide benzoïque au mélange de l'agent augmentant la viscosité et de glycérol, tout en agitant ledit mélange pour former une solution du sel d'acide benzoïque physiologiquement acceptable dans le milieu aqueux ;
e. facultativement laisser le milieu aqueux gonfler, tout en agitant le milieu aqueux ;
dans lequel le sel d'acide benzoïque physiologiquement acceptable a une concentration d'au moins 1,39 mmol/ml dans le milieu aqueux, et dans lequel le glycérol a une concentration volumique d'au moins 1,0 ml/l par rapport au volume total du milieu aqueux.

9. Procédé permettant de préparer la composition liquide pharmaceutique selon la revendication 8, dans lequel :
la quantité d'agent augmentant la viscosité ajouté au glycérol pendant l'étape b. est choisie de telle sorte que la concentration de l'agent augmentant la viscosité dans le mélange résultant est comprise dans la plage de 0,01 g/ml à 0,10 g/ml, de préférence dans la plage de 0,02 g/ml à 0,08 g/ml ; et/ou
l'agent augmentant la viscosité est dispersé dans du glycérol lors de l'ajout de la solution du sel d'acide benzoïque physiologiquement acceptable au mélange d'agent augmentant la viscosité et de glycérol.

10. Composition liquide pharmaceutique selon la revendication 1 ou trousse de parties selon la revendication 5 pour une utilisation en tant que médicament, de préférence pour la prévention et/ou le traitement d'un trouble génétique d'un patient.

11. Composition liquide pharmaceutique pour une utilisation selon la revendication 10, dans laquelle la composition liquide pharmaceutique est administrée à l'aide d'une sonde nasogastrique et/ou gastrique.

12. Composition liquide pharmaceutique pour une utilisation selon la revendication 10, dans laquelle la solution liquide pharmaceutique est administrée à un patient qui en a besoin, l'administration comprenant :
a. l'ajout d'une quantité de la composition liquide pharmaceutique à de l'eau pour former un mélange, dans laquelle le rapport volumique entre la composition liquide pharmaceutique et l'eau est compris dans la plage de 1:20 à 1:1, de préférence dans la plage de 1:10 à 1:2.
b. par voie orale, de préférence quotidiennement, l'administration dudit mélange au patient ;
dans laquelle, facultativement, la quantité de la composition liquide pharmaceutique ajoutée à l'étape a. à de l'eau est comprise dans la plage de 0,5 ml à 20,0 ml.

13. Composition liquide pharmaceutique pour une utilisation selon la revendication 10, 11 ou 12, dans laquelle la composition liquide pharmaceutique est utilisée pour une administration quotidienne au patient, de préférence en une dose d'une quantité thérapeutiquement efficace de la composition liquide pharmaceutique, plus préférablement en trois doses liquides divisées de manière égale, ou plus.

14. Formulation liquide pharmaceutique pour une utilisation selon la revendication 12, dans laquelle la composition liquide pharmaceutique fait partie d'une trousse de parties selon la revendication 4, le procédé comprenant en outre une étape avant l'étape d'administration orale consistant à ;
c. ajouter une quantité dudit concentré liquide aromatisant, comprise de préférence dans la plage de 0,01 ml à 1,00 ml, de préférence dans la plage de 0,02 ml à 0,50 ml, plus préférablement dans la plage de 0,02 ml à 0,25 ml au mélange de l'étape a. ou à l'eau, qui est utilisée à l'étape a. pour former le mélange ;
dans laquelle, facultativement, la trousse de parties comprend au moins deux concentrés liquides aromatisants et dans laquelle l'étape consistant à ajouter un ou plusieurs concentrés liquides aromatisants comprend la sélection d'une quantité de gouttes d'un premier concentré liquide aromatisant et la sélection d'une quantité de gouttes d'un second concentré liquide aromatisant.
